# EUROPEAN PATENT SPECIFICATION

(11) **EP 2 365 792 B1**
(45) Date of publication and mention of the grant of the patent: **11.12.2013**
(21) Application number: 09741144.1
(22) Date of filing: 16.10.2009
(51) Int. Cl.: A61F 2/38

(54) **TIBIAL TRAY HAVING A REINFORCING MEMBER**
TIBIALSCHALE MIT VERSTÄRKUNGSELEMENT
PLATEAU TIBIAL COMPORTANT UN ÉLÉMENT DE RENFORT

(30) Priority: 17.10.2008 US 253259; 15.10.2009 US 579479
(43) Date of publication of application: 21.09.2011
(73) Proprietor: Biomet Manufacturing, LLC, Warsaw, IN 46582 (US)
(72) Inventor: MAY, Brian M., Warsaw Indiana 46582 (US); KUMAR, Mukesh, Warsaw Indiana 46582 (US)
(74) Representative: Giles, Ashley Simon
(86) International application number: PCT/US2009/060978
(87) International publication number: WO 2010/045537

(56) References cited:
- WO-A-00/25700
- DE-A1- 3 136 636
- DE-A1- 19 705 733
- US-A- 5 534 027

## Description

### CROSS-REFERENCE TO RELATED APPLICATIONS

This application claims priority to U.S. Serial No. 12/579,479, file on October 15, 2009 which is a continuation-in-part of United States Patent Application No. 12/253,259 filed on October 17, 2008.

### FIELD

The present disclosure relates to tibial trays and more particularly to a tibial tray incorporating a reinforcing member.

### BACKGROUND

The statements in this section merely provide background information related to the present disclosure and may not constitute prior art.

In some instances, the knee joint may undergo degenerative changes due to multiple etiologies. In some examples, when these degenerative changes are advanced, irreversible and unresponsive to non-operative management, it may ultimately become necessary to replace some or all of the natural knee joint with knee joint prosthetics. In one example, a knee joint prosthesis can comprise a femoral component and a tibial component. The femoral component and the tibial component can be designed to be surgically attached to the distal end of the femur and the proximal end of the tibia, respectively. The femoral component can further be designed to cooperate with the tibial component in simulating the articulating motion of an anatomical knee joint.

Typically, the tibial component can include a substantially planar platform-like tibial tray and an inferiorly extending tibial stem. The tibial stem can be adapted to be received in a corresponding opening made by a surgeon in the longitudinal center of the tibia. In general, it can be desired to provide a tibial tray having sufficient fatigue strength as well as providing an area for bone fixation.

DE3136636 discloses a tibial plateau as a knee joint endoprosthesis comprising a plastic part forming the actual plateau and a baseplate of hard material such as metal. Connection of these parts is achieved by a collar provided on the baseplate with an undercut connecting surface, and an interacting step on the plateau with a connecting surface, and by an engagement means which holds the connecting surfaces against one another.

US5534027 discloses an orthopaedic implant assembly including a securing member or screw and a sealing washer assembled thereto. The assembly further includes a base member with a hole therein for receiving the screw and the washer. The screw includes an enlarged head with an accurate undersurface with a groove surrounding head for receiving the sealing washer therein.

### SUMMARY

A knee joint prosthesis according to the invention is defined in claim 1.

According to additional features. The tibial component can further comprise a stem that extends inferiorly from the platform-like tray. According to one example, the platform-like tray and the raised wall can be integrally formed of solid biocompatible material. The raised wall can be adapted to engage cancellous bone and the porous material can be adapted to engage cortical bone in an implant position.

According to additional features, the porous material can define a first thickness at the tray perimeter and a second thickness at the raised wall. The second thickness can be greater than the first thickness. According to one example; the platform-like tray can further comprise a first support wall formed on the inferior surface and extending between a generally anterior/lateral position to a posterior/medial position. The platform-like tray can also include a second support wall formed on the inferior surface and extending between a generally anterior/medial position to a posterior/lateral position. According to various examples, the first and second support walls can define a generally dove-tail cross-section. The knee joint prosthesis can additionally comprise a femoral component and a bearing selectively coupled to the tibial component.

Further areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.

Fig. 1 is an anterior perspective view of an exemplary tibial tray constructed in accordance with one example of the present teachings;

Fig. 2 is an inferior view of the tibial tray of Fig. 1;

Fig. 3 is a cross-sectional view of the tibial tray of Fig. 2 taken along line 3-3 and shown implanted into an exemplary tibia;

Fig. 4A is a cross-sectional view of a tibial tray constructed in accordance to additional features of the present disclosure;

Fig. 4B is a cross-sectional view of a tibial tray constructed in accordance to additional features of the present disclosure;

Fig. 5 is an inferior view of another tibial tray constructed in accordance to additional features of the present disclosure;

Fig. 6 is a cross-sectional view of the tibial tray of Fig. 5 and taken along line 6-6;

Fig. 7 is an inferior view of a tibial tray constructed in accordance to additional features of the present disclosure;

Fig. 8 is a cross-sectional view of the tibial tray of Fig. 7 and taken along line 8-8;

Fig. 9 is a cross-sectional view of another tibial tray illustrating a support wall having an alternate cross-section;

Fig. 10 is a cross-sectional view of yet another tibial tray illustrating a support wall having an alternate cross-section;

Fig. 11 is an exploded anterior view of a knee joint prosthesis including the tibial tray shown in Fig. 1 according to the present disclosure; and

Fig. 12 is an anterior perspective view of the knee joint prosthesis of Fig. 11 and shown in an implanted position with a surgically prepared femur and tibia according to the present disclosure.

### DETAILED DESCRIPTION

The following description is merely exemplary in nature and is not intended to limit the present disclosure, its application, or uses.

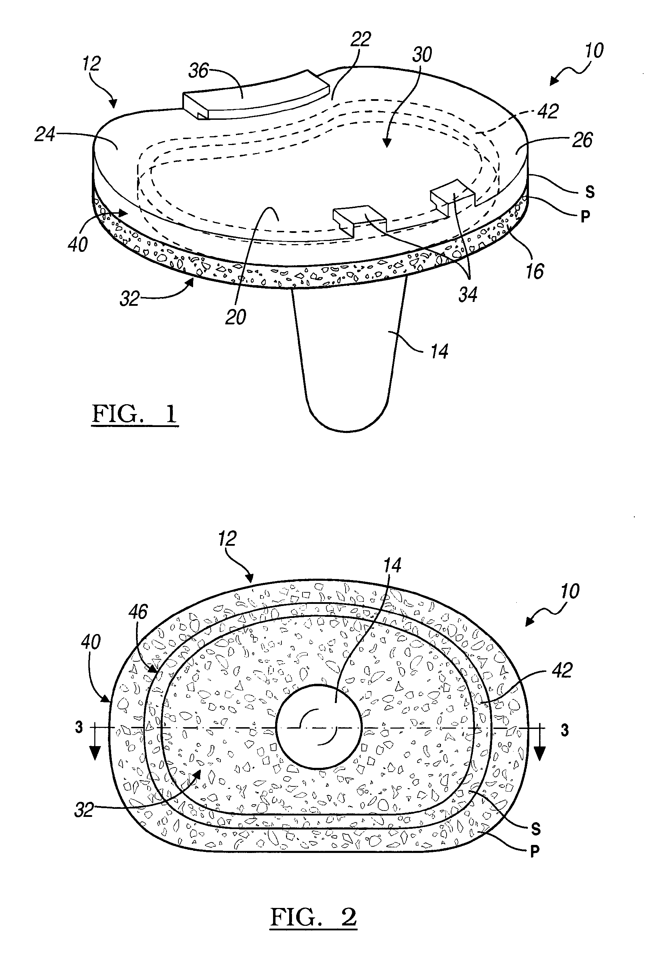

With initial reference now to Fig. 1, a tibial component constructed according to the present teachings is shown and generally identified at reference numeral 10. The tibial component 10 generally includes a substantially planar platform-like tray 12 having a modular, inferiorly extending tibial stem 14. In other examples, the stem 14 can be integrally formed with the tibial tray 12. The tibial stem 14 can be adapted to be received in a corresponding opening made by a surgeon in a proximal tibia. The tibial component 10 according to the present teachings incorporates porous material 16 at selected areas as will be described. As will become appreciated from the following discussion, the tibial component 10 constructed in accordance to the present teachings can increase a fatigue strength of the tibial tray 12 while simultaneously maximizing the volume of porous material 16 intended for bone fixation. The tibial tray 12 can provide a solid substrate portion S and a porous material portion P.

For discussion purposes, the tibial component 10 will be described for use with a knee joint having a surgically resected left tibia. It is understood, however, that the tibial component 10 may be universal, such that it may be adapted for use with a surgically resected right tibia. Likewise, the tibial component 10 may be adapted for use in either a left or a right tibia. The tibial tray 12 can generally define an anterior portion 20, a posterior portion 22, a medial portion 24, and a lateral portion 26. The tibial tray 12 generally defines a superior bearing engaging surface 30 and an inferior bone engaging surface 32 (see also Fig. 2).

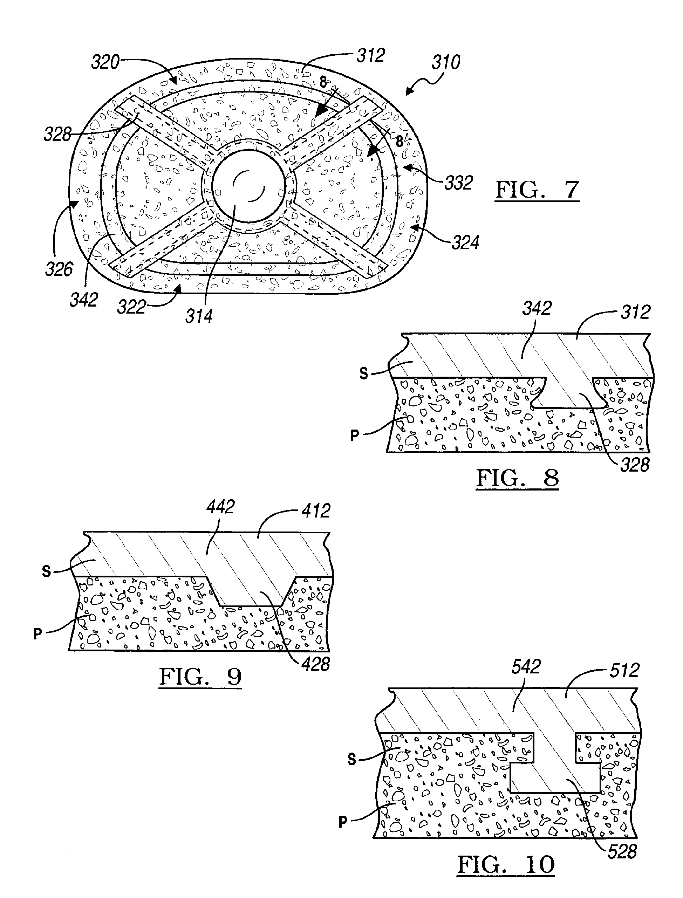

The exemplary tibial tray 12 can define a pair of integrally formed posts 34, which extend superiorly at the anterior portion 20. A catch 36 can also be defined at the posterior portion 22. The posts 34 may cooperate with a locking bar (not specifically shown) to secure a tibial bearing 38 (Figs. 9 and 10). In this way, the posts 34 and catch 36 can be used to secure a tibial bearing 38 to the tibial tray 12. It is appreciated that other retaining features may be employed for securing a tibial bearing 38 to the tibial tray 12. Likewise, it is appreciated, that the tibial tray 12 may alternatively be adapted for use with a floating bearing. In such an example, the superior surface 30 may be highly polished to provide a substantially smooth tibial bearing surface. While not specifically shown, a floating bearing having a substantially planar inferior bearing surface may be located above the tibial tray 12. In this way, the floating bearing may slidably move relative to the highly polished superior surface 30 of tibial tray 12. The tibial tray 12 may be adapted for use in a cruciate retaining (CR) knee replacement, a posterior stabilized (PS) knee replacement and a fully constrained knee replacement for example.

With continued reference to Fig. 1 and additional reference now to Figs. 2-4, additional features of the tibial tray 12 will be described in greater detail. In general, the tibial tray 12 defines a tray perimeter 40 and a raised wall 42. The raised wall 42 is a closed wall formed around the inferior surface 32 of the tibial tray 12. In one example, the raised wall 42 can be integrally formed with the tibial tray 12 as a whole. The tibial tray 12 and the raised wall 42 can collectively, define the solid substrate portion S. Explained differently, the raised wall 42 can be monolithic or one-piece with the tibial tray 12. In one example, the solid substrate portion S, including the tray 12 and the raised wall 42 (and in some examples, the stem 14) can be formed of solid biocompatible material, such as, but not limited to titanium. In other embodiments, non-metal materials may be employed for the substrate, such as, but not limited to, polyetheretherketone (PEEK), fiber-reinforced PEEK, and ceramics. The solid biocompatible material portions of the tray 12 can be formed by any suitable means, such as by machining, molding, casting or other methods.

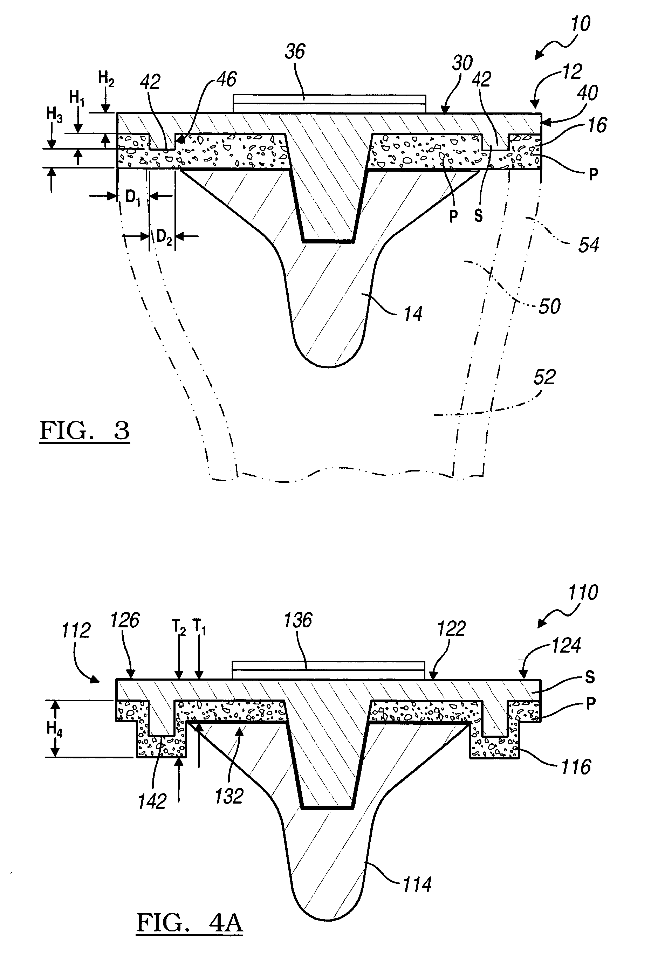

According to the present teachings, the raised wall 42 is offset inboard relative to the tray perimeter 40. As will be described in more detail, the raised wall 42 can be offset inboard a suitable distance to be aligned with a cancellous bone 50 of a tibia 52. The porous material portion 16 is arranged between the raised wall 42 and the tray perimeter 40 (Fig. 3). The configuration of the tibial tray 12 provides porous material 16 outboard of the raised wall 42 in areas that can be aligned with cortical bone 54 of the tibia 52. As best shown in Fig. 2, the raised wall 42 can define a wall perimeter 46 that is stepped inboard (such as in a direction toward the stem 14), a substantially equivalent distance around the inferior surface 32 of the tibial tray 12 relative to the tray perimeter 40. Furthermore, the raised wall 42 is formed inboard to provide enhanced mechanical strength (such as tensile strength and/or rigidity) to a tray that can be formed thinner versus a tray without a ridge.

With specific reference now to Fig. 3, exemplary dimensions of the tibial tray 12 will be described. In one example, the raised wall 42 can be offset inboard relative to the tray perimeter 40 a distance D₁ to provide mechanical strength (such as a sufficient stiffness or tensile strength). The raised wall 42 can define a lateral thickness D₂. In one example, D₁ can be about 2-3mm. It is appreciated that D₁ can vary slightly (i.e., the distance between the tray perimeter 40 and the wall perimeter 46) around the tibial tray 12. The distance D₂ can be about 2-4mm. The porous material 16 can define a total height of height H₁ plus height H₃. In one example, the height H₁ can be substantially equivalent to a height of the raised wall 42. The height H₁ plus H₃ can be greater than a thickness of the solid substrate portion H₂ of the tibial tray 12. In one exemplary configuration, H₂ is about 1.5mm, H₁ is about 1 mm and H₃ is about 1.5mm. Again, those skilled in the art will appreciate that these dimensions are merely exemplary. These dimensions can be optimized, such that the raised wall 42 can be positioned at an area suitable to cooperate with the cancellous bone 50 of a particular patient. Consequently, the porous material 16 can be optimized to interface with the cortical bone 54 of a particular patient.

In general, maximum flexion of the tibial tray 12 can occur at its periphery. When implanted, this flexion generally does not materialize due the support of the surrounding cortical bone 50 of the tibia 52. The configuration of the raised wall 42 and the porous material 16 according to the present teachings can allow for deeper bone ingrowth due to maximum loading of the surrounding cortical bone 54. In one example, the configuration and placement of the raised wall 42 can allow for thinner thicknesses of the tibial tray 12 because of the increased rigidity the raised wall 42 can provide. It has been shown through load testing that a tibial tray having a raised wall positioned inboard from a periphery of the tray (such as the tibial tray 10, or others disclosed herein) experiences a reduction of principle stress of at least 30% and about 45% as compared to a tibial tray having a raised wall formed on its periphery (given the same load).

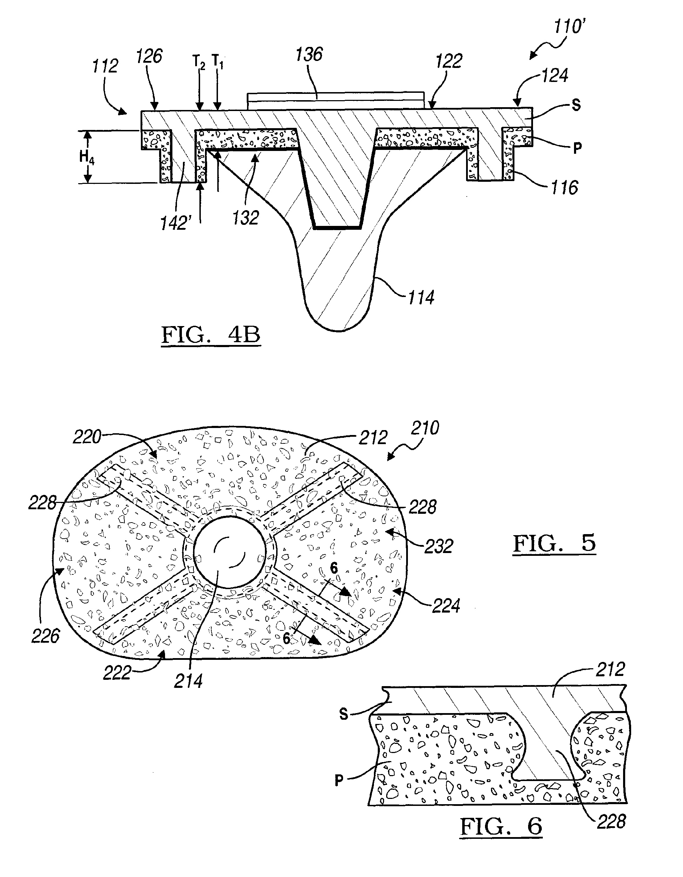

Turning now to Fig. 4A, a tibial component 110 constructed in accordance to additional features is shown. The tibial component 10 can include a substantially planar platform-like tibial tray 112 having an inferiorly extending tibial stem 114. The tibial component 110 can incorporate porous material 116. The tibial tray 112 can provide a solid substrate portion S and a porous material portion P. The tibial tray 112 can define a medial portion 124 and a lateral portion 126. A catch 136 can be defined on a posterior portion 122. A raised wall 142 can be defined around an inferior surface 132 of the tibial tray 112 similar to that described above with respect to the tibial tray 12. In the tibial tray 112, shown in Fig. 4A, the raised wall 142 and the porous material 116 can collectively define a height H₄. The height H₄ can be greater than the height H₁ plus H₃ described with respect to the tray 12 (see Fig. 3). In one example, the height H₄ can be between 2-4mm. As can be appreciated, the raised wall 142 having an increased height of H₄ can provide increased mechanical strength (i.e., fatigue and/or tensile strength) of the tray 112 as a whole. As shown in Fig. 4A, the porous material 116 can have a first thickness T₁ and a second thickness T₂. In one example, the thickness T₁ can be substantially about 2-4mm and the thickness T₂ can be substantially about 4-6mm. Again, those skilled in the art will appreciate that these diameters are merely exemplary. Other dimensions and ranges are contemplated. A tibial component 110' shown in Fig. 4B is constructed in accordance to additional features. The tibial component 110' is constructed similar to the tibial component 110 (Fig. 4A) except a raised wall 142' extends the length of H₄ (i.e., no porous material 116 is provided on an inferior surface of the raised wall 142').

The porous material 16, 116 can be any metal or alloy that is suitable for use in an implant and provide the desired strength and load bearing capability according to a particular application. Suitable exemplary metals can include titanium, cobalt, chromium, or tantalum, alloys thereof, stainless steel, and combinations thereof. One suitable porous metal and method for making the same may be found in commonly owned and copending U.S. Serial No. 11/357,929, filed February 17, 2006, entitled "Method and Apparatus for Forming Porous Metal Implants".

The porous material portion P (i.e., porous material 16, 116) can be attached to the solid substrate portion S of the tibial tray 12 by any suitable means, such as welding, sintering, using a laser, etc. In various embodiments, the solid substrate portion S of the tibial tray 12 can be formed of metal, such as the same metal as the porous material portion P. The solid substrate portion S of the tibial tray 12 can be prepared prior to attaching the porous material portion P. The solid substrate portion S of the tibial tray 12 can be acid etched, subjected to an acid bath, grit blasted, or ultrasonically cleaned for example. Other preparations can include adding channels, pits, grooves, indentations, bridges, or holes into the solid substrate portion S of the tibial tray 12. These additional features may increase the attachment of the porous portion P to the solid substrate portion S of the tibial tray 12.

Additional agents can be coated onto or in at least a surface of the porous material 16. Agents can include resorbable ceramics, resorbable polymers, antibiotics, demineralized bone matrix, blood products, platelet concentrate, allograft, xenograft, autologous and allogeneic differentiated cells or stem cells, nutrients, peptides and/or proteins, vitamins, growth factors, and mixtures thereof, which would facilitate ingrowth of new tissue into the porous material 16.

With reference now to Fig. 5, a tibial component 210 constructed not in accordance is shown. The tibial component 210 can include a substantially planar platform-like tibial tray 212 having an inferiorly extending tibial stem 214. The tibial tray 212 can define an anterior portion 220, a posterior portion 222, a medial portion 224, and a lateral portion 226. A pair of support walls 228 can be defined across the inferior surface 232 of the tibial tray 212. According to the example shown, one of the support walls 228 can extend from an anterior/lateral position to a posterior/medial position. The other support wall 228 can extend from an anterior/medial position to a posterior/lateral position. As best shown in Fig. 6, a cross-section of the support wall 228 can define a generally dove-tail shape. It is appreciated that the raised wall 42 (Fig. 2) and/or 142 (Fig. 4) can define a dove-tail shape. The tibial tray 212 can provide a solid substrate portion S and a porous material portion P. In one example, such as shown in Fig. 6, the porous material portion P can extend inferiorly beyond the support wall 228. In another example, the porous material portion P may not extend inferiorly beyond the support wall 228 (such as shown in Fig. 3).

Turning now to Figs. 7 and 8, a tibial component 310 constructed in accordance to additional features is shown. The tibial component 310 can include a substantially planar platform-like tibial tray 312 having an inferiorly-extending tibial stem 314. The tibial tray 312 can define an anterior portion 320, a posterior portion 322, a medial portion 324, and a lateral portion 326. The tibial tray 312 of Fig. 7 can define a pair of support walls 328, such as described above in relation to the tibial tray 212 of Fig. 5. The tibial tray 312 can further define a raised wall 342. The raised wall 342 can be constructed, such as described above with respect to the raised wall 42 of the tibial tray 12 (Figs. 1-3). According to some examples, the relative offsets and thicknesses of the raised wall 342 and the porous material portion P can be similar to those described in relation to Figs. 3 and 4 above. Fig. 9 illustrates a tibial tray 412 having a raised wall 442 that includes support walls 428. Fig. 10 illustrates a tibial tray 512 having a raised wall 542 that includes support walls 528.

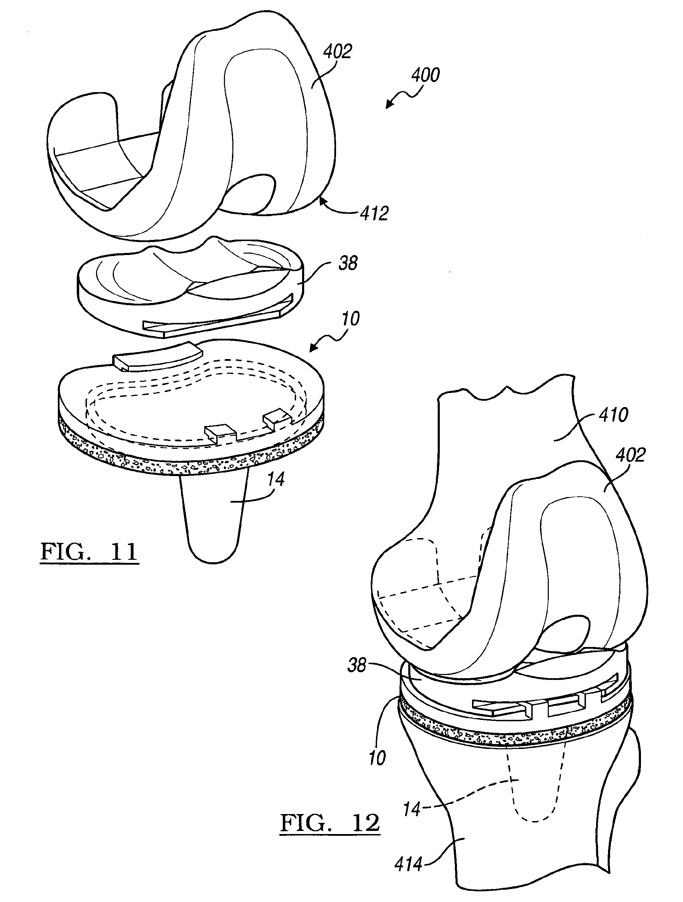

With reference now to Figs. 11 and 12, the tibial component (or 110, 210, or 310) can be used as part of a total knee prosthesis 400. In one example, the total knee prosthesis 400 can include a femoral component 402, the tibial component 10, and the bearing 38. As is known, the femoral component 402 can be rigidly connected to a distal end of a femur 410 (Fig. 12) after the femur 410 has been resected in a manner, which is well known in the art. The femoral component 402 can include a condylar portion 412, which engages the bearing 38. The tibial component 10 can be connected to a tibia 414 (Fig. 12) by any suitable method. The bearing 38 can be made from any suitable material, such as ultra-high molecular weight polyethylene (UHMWP). The total knee prosthesis 400 can be part of any knee joint, such as, but not limited to, cruciate retaining (CR), posterior stabilized (PS), and fully constrained (FC).

Those skilled in the art can now appreciate from the foregoing description that the broad teachings of the present disclosure can be implemented in a variety of forms. Therefore, while this disclosure has been described in connection with particular examples thereof, the true scope of the disclosure should not be so limited since other modifications will become apparent to the skilled practitioner upon a study of the drawings, the specification and the following claims.

## Claims

1. A knee joint prosthesis comprising:
a tibial component (10) including a tibial tray, said tibial tray comprising:
a platform-like tray (12) defining a superior surface (30) and an inferior surface (32), said platform-like tray (12) defining a tray perimeter;
a raised wall (42) formed on and extending from said inferior surface (32) of said platform-like tray (12), said raised wall (42) offset inboard relative to said tray perimeter (40) and defining a closed wall having a profile that is substantially equivalent to said tray perimeter (40) around said inferior surface (32) and configured to provide increased rigidity of said platform-like tray (12); and
porous material (16) disposed on said inferior surface (32) of said platform-like tray (12) at a location generally between said raised wall (42) and said tray perimeter (40).

2. The knee joint prosthesis of claim 1 wherein said platform-like tray (12) has a first height measured between said superior surface (30) and said inferior surface (32), said porous material (16) having a second height measured between said inferior surface (32) and a bone engaging surface of said porous material (16), said second height being greater than said first height.

3. The knee joint prosthesis of claim 1 wherein said tibial component further comprises, a stem (14) that extends inferiorly from said platform-like tray (12).

4. The knee joint prosthesis of claim 3 wherein said porous material (16) is disposed in a location generally between said raised wall (42) and said stem (14).

5. The knee joint prosthesis of claim 1 wherein said platform-like tray (12) and said raised wall (42) are integrally formed of solid biocompatible material selected from the group comprising metal, PEEK, fiber-reinforced PEEK and ceramic.

6. The knee joint prosthesis of claim 1 wherein said raised wall (42) is perpendicular relative to a plane define by said platform-like tray (12) and is adapted to be aligned with cancellous bone and said porous material (16) is adapted to abut cortical bone in an implanted position.

7. The knee joint prosthesis of claim 6 wherein said porous material (16) defines a first thickness at said tray perimeter (40) and a second thickness at said raised wall (42), wherein said second thickness is greater than said first thickness.

8. The knee joint prosthesis of claim 1 wherein said platform-like tray (12) further comprises, a first support wall (228) formed on said inferior surface (32) and extending between a generally anterior/lateral position to a posterior/medial position.

9. The knee joint prosthesis of claim 7 wherein said platform-like tray (12) further comprises, a second support wall (228) formed on said inferior surface (32) and extending between a generally anterior/medial position to a posterior/lateral position.

10. The knee joint prosthesis of claim 9 wherein said first and second support walls (228) define a generally dove-tail cross-section.

11. The knee joint prosthesis of claim 1, further comprising:
a femoral component (402); and
a bearing (38) selectively engageable with said tibial component (10).

12. The knee joint prosthesis of claim 1, wherein:
said tibial tray comprises a solid metal portion comprising a stem (14) extending inferiorly from said platform-like tray (12);
the raised wall (42) extends to a bone engaging end face, said raised wall (42) being offset toward said stem (14), and
the porous material (16) comprises:
a first porous metal portion disposed on said inferior surface (32) outboard of said raised wall (42);
a second porous metal portion disposed on said inferior surface (32) inboard of said raised wall (42), wherein said first and second porous metal portions are discontinuous at said bone engaging end face such that said bone engaging end face is free of porous metal and configured to directly engage bone.

13. The knee joint prosthesis of claim 12 wherein said raised wall (42) is adapted to be aligned with cancellous bone and said first porous metal portion is adapted to abut cortical bone in an implanted position.

14. The knee joint prosthesis of claim 12 wherein said platform-like tray (12) further comprises, first and second support walls (228) formed on said inferior surface (32) and extending radially therealong.

15. The knee joint prosthesis of claim 14 wherein at least one of said first and second support walls (228) defines a generally dove-tail cross-section.

16. The knee joint prosthesis of claim 12, further comprising:
a femoral component (402); and
a bearing (38) selectively coupled to said tibial component (10).

## Patentansprüche

1. Kniegelenksprothese, umfassend:
eine Tibiakomponente (10), einschließlich eines Tibiaplateaus, wobei das Tibiaplateau umfasst:
ein plattformartiges Plateau (12), das eine Oberseite (30) und eine Unterseite (32) definiert, wobei das plattformartige Plateau (12) einen Plateau-Umkreis definiert; eine emporragende Wand (42), die auf der Unterseite (32) des plattformartigen Plateaus (12) gebildet ist und von dort ausgeht, wobei die emporragende Wand (42) innen zum Plateau-Umkreis (40) versetzt ist und eine geschlossene Wand mit einem Profil definiert, das im Wesentlichen zu dem Plateau-Umkreis (40) um die Unterseite (32) äquivalent ist und so konfiguriert ist, dass das plattformartige Plateau (12) eine größere Festigkeit erhält; und
poröses Material (16), das auf der Unterseite (32) des plattformartigen Plateaus (12) an einer im Allgemeinen zwischen der emporragenden Wand (42) und dem Plateau-Umkreis (40) befindlichen Stelle angeordnet ist.

2. Kniegelenksprothese nach Anspruch 1, wobei das plattformartige Plateau (12) eine erste Höhe aufweist, die zwischen der Oberseite (30) und der Unterseite (32) gemessen wird, und das poröse Material (16) eine zweite Höhe aufweist, die zwischen der Unterseite (32) und einer knochengreifenden Fläche des porösen Materials (16) gemessen wird, wobei die zweite Höhe größer als die erste Höhe ist.

3. Kniegelenksprothese nach Anspruch 1, wobei die Tibiakomponente zudem einen Stiel (14) umfasst, der von dem plattformartigen Plateau (12) nach unten ragt.

4. Kniegelenksprothese nach Anspruch 3, wobei das poröse Material (16) an einer im Allgemeinen zwischen der emporragenden Wand (42) und dem Stiel (14) befindlichen Stelle angeordnet ist.

5. Kniegelenksprothese nach Anspruch 1, wobei das plattformartige Plateau (12) und die emporragende Wand (42) einstückig aus einem festen biokompatiblen Material herausgearbeitet sind, das aus der Gruppe, die Metall, PEEK, faserverstärktes PEEK und Keramik umfasst, ausgewählt ist.

6. Kniegelenksprothese nach Anspruch 1, wobei die emporragende Wand (42) senkrecht zu einer Ebene ist, die von dem plattformartigen Plateau (12) definiert wird und zu einem spongiösen Knochen ausgerichtet werden kann, und das poröse Material (16) in einer implantierten Position an den kortikalen Knochen stoßen kann.

7. Kniegelenksprothese nach Anspruch 6, wobei das poröse Material (16) eine erste Dicke an dem Plateau-Umkreis (40) definiert und eine zweite Dicke an der emporragenden Wand (42) definiert, wobei die zweite Dicke größer ist als die erste Dicke.

8. Kniegelenksprothese nach Anspruch 1, wobei das plattformartige Plateau (12) zudem eine auf der Unterseite (32) gebildete erste Stützwand (228) umfasst, die zwischen einer im Allgemeinen anterioren/lateralen Position zu einer posterioren/medialen Position verläuft.

9. Kniegelenksprothese nach Anspruch 7, wobei das plattformartige Plateau (12) zudem eine auf der Unterseite (32) gebildete zweite Stützwand (228) umfasst, die zwischen einer im Allgemeinen anterioren/medialen Position zu einer posterioren/lateralen Position verläuft.

10. Kniegelenksprothese nach Anspruch 9, wobei die ersten und zweiten Stützwände (228) einen im Allgemeinen schwalbenschwanzförmigen Querschnitt definieren.

11. Kniegelenksprothese nach Anspruch 1, zudem umfassend:
eine Femurkomponente (402); und
ein Lager (38), das selektiv in die Tibiakomponente eingreifen kann.

12. Kniegelenksprothese nach Anspruch 1, wobei:
das Tibiaplateau einen festen Metallteil umfasst, der einen von dem plattformartigen Plateau (12) nach unten verlaufenden Stiel (14) aufweist;
die emporragende Wand (42) zu einer knochengreifenden Endfläche verläuft, wobei die emporragende Wand (42) zum Stiel (14) versetzt ist, und
das poröse Material (16) umfasst:
einen ersten porösen Metallteil, der auf der Unterseite (32) außerhalb der emporragenden Wand (42) angeordnet ist;
einen zweiten porösen Metallteil, der auf der Unterseite (32) innerhalb der emporragenden Wand (42) angeordnet ist, wobei die ersten und zweiten porösen Metallteile an der knochengreifenden Endfläche unterbrochen sind, so dass die knochengreifende Endfläche frei von porösem Metall ist, und sie so konfiguriert ist, dass sie den Knochen direkt greift.

13. Kniegelenksprothese nach Anspruch 12, wobei die emporragende Wand (42) zu einem spongiösen Knochen ausgerichtet werden kann, und der erste poröse Metallteil in einer implantierten Position an den kortikalen Knochen stoßen kann.

14. Kniegelenksprothese nach Anspruch 12, wobei das plattformartige Plateau (12) zudem erste und zweite Stützwände (228) umfasst, die auf der Unterseite (32) gebildet sind, und dort entlang radial verlaufen.

15. Kniegelenksprothese nach Anspruch 14, wobei mindestens eine der ersten und zweiten Stützwände (228) einen im Allgemeinen schwalbenschwanzförmigen Querschnitt definiert.

16. Kniegelenksprothese nach Anspruch 12, zudem umfassend:
eine Femurkomponente (402); und
ein Lager (38), das selektiv an die Tibiakomponente (10) gekoppelt ist.

## Revendications

1. Prothèse de l'articulation du genou, comprenant :
un composant tibial (10) incluant un plateau tibial, ledit plateau tibial comprenant :
un plateau semblable à une plateforme (12) définissant une surface supérieure (30) et une surface inférieure (32), ledit plateau semblable à une plateforme (12) définissant un périmètre de plateau ;
une paroi relevée (42) formée sur et s'étendant de ladite surface inférieure (32) dudit plateau semblable à une plateforme (12), ladite paroi relevée (42) étant décalée vers l'intérieur relativement audit périmètre de plateau (40) et définissant une paroi fermée ayant un profile qui est sensiblement équivalent audit périmètre de plateau (40) autour de ladite surface inférieure (32) et configurée pour conférer une plus grande rigidité audit plateau semblable à une plateforme (12) ; et
un matériau poreux (16) disposé sur ladite surface inférieure (32) dudit plateau semblable à une plateforme (12) à un emplacement généralement entre ladite paroi relevée (42) et ledit périmètre de plateau (40).

2. Prothèse de l'articulation du genou selon la revendication 1, dans laquelle ledit plateau semblable à une plateforme (12) a une première hauteur mesurée entre ladite surface supérieure (30) et ladite surface inférieure (32), ledit matériau poreux (16) ayant une deuxième hauteur mesurée entre ladite surface inférieure (32) et une surface venant en prise avec l'os dudit matériau poreux (16), ladite deuxième hauteur étant plus grande que ladite première hauteur.

3. Prothèse de l'articulation du genou selon la revendication 1, dans laquelle ledit composant tibial comprend en outre une tige (14) qui s'étend côté inférieur dudit plateau semblable à une plateforme (12).

4. Prothèse de l'articulation du genou selon la revendication 3, dans laquelle ledit matériau poreux (16) est disposé dans un emplacement généralement entre ladite paroi relevée (42) et ladite tige (14).

5. Prothèse de l'articulation du genou selon la revendication 1, dans laquelle ledit plateau semblable à une plateforme (12) et ladite paroi relevée (42) sont réalisés intégralement en un matériau biocompatible solide sélectionné dans le groupe comprenant le métal, PEEK, PEEK renforcé par des fibres et céramique.

6. Prothèse de l'articulation du genou selon la revendication 1, dans laquelle ladite paroi relevée (42) est perpendiculaire relativement à un plan défini par ledit plateau semblable à une plateforme (12) et est apte à être alignée avec l'os spongieux, et ledit matériau poreux (16) est apte à buter contre l'os cortical dans une position implantée.

7. Prothèse de l'articulation du genou selon la revendication 6, dans laquelle ledit matériau poreux (16) définit une première épaisseur audit périmètre de plateau (40) et une deuxième épaisseur à ladite paroi relevée (42), où ladite deuxième épaisseur est plus grande que ladite première épaisseur.

8. Prothèse de l'articulation du genou selon la revendication 1, dans laquelle ledit plateau semblable à une plateforme (12) comprend en outre une première paroi de support (228) formée sur ladite surface inférieure (32) et s'étendant entre une position généralement antérieure/latérale à une position postérieure/médiale.

9. Prothèse de l'articulation du genou selon la revendication 7, dans laquelle ledit plateau semblable à une plateforme (12) comprend en outre une deuxième paroi de support (228) formée sur ladite surface inférieure (32) et s'étendant entre une position généralement antérieure/médiale à une position postérieure/latérale.

10. Prothèse de l'articulation du genou selon la revendication 9, dans laquelle lesdites première et deuxième parois de support (228) définissent une section transversale généralement en forme de queue d'aronde.

11. Prothèse de l'articulation du genou selon la revendication 1, comprenant en outre :
un composant fémoral (402) ; et
un support (38) pouvant être sélectivement mis en prise avec ledit composant tibial.

12. Prothèse de l'articulation du genou selon la revendication 1, dans laquelle :
ledit plateau tibial comprend une portion métallique solide comprenant une tige (14) s'étendant au côté inférieur depuis ledit plateau en forme de plateforme (12) ;
la paroi relevée (42) s'étend à une face d'extrémité de mise en prise avec l'os, ladite paroi relevée (42) étant décalée vers ladite tige (14), et
le matériau poreux (16) comprend :
une première portion métallique poreuse disposée sur ladite surface inférieure (32) à l'extérieur de ladite paroi relevée (42) ;
une deuxième portion métallique poreuse disposée sur ladite surface inférieure (32) à l'intérieur de ladite paroi relevée (42), où lesdites première et deuxième portions métalliques poreuses sont discontinues à ladite face d'extrémité de mise en prise avec l'os de telle sorte que ladite face d'extrémité de mise en prise avec l'os est exempte de métal poreux et est configurée pour venir directement en prise avec l'os.

13. Prothèse de l'articulation du genou selon la revendication 12, dans laquelle ladite paroi relevée (42) est apte à être alignée avec l'os spongieux, et ladite première portion métallique poreuse est apte à buter contre l'os cortical dans une position implantée.

14. Prothèse de l'articulation du genou selon la revendication 12, dans laquelle ledit plateau semblable à une plateforme (12) comprend en outre des première et deuxième parois de support (228) formées sur ladite surface inférieure (32) et s'étendant radialement le long de celle-ci.

15. Prothèse de l'articulation du genou selon la revendication 14, dans laquelle au moins une parmi lesdites première et deuxième parois de support (228) définit une section transversale généralement en queue d'aronde.

16. Prothèse de l'articulation du genou selon la revendication 12, comprenant en outre :
un composant fémoral (402) ; et
un support (38) sélectivement couplé audit composant tibial (10).
